# FASCICULE DE BREVET EUROPEEN

(11) **EP 2 089 720 B1**
(45) Date de publication et mention de la délivrance du brevet: **12.04.2017**
(21) Numéro de dépôt: 07870293.3
(22) Date de dépôt: 16.11.2007
(51) Int. Cl.: G01N 33/68

(54) **UTILISATION COSMETIQUE D'UNE PROTEINE DE LA FAMILLE DES RIBONUCLEASES**
KOSMETISCHE VERWENDUNG EINES ZUR RIBONUKLEASE-FAMILIE<0} GEHÖRENDEN PROTEINS
COSMETIC USE OF A PROTEIN BELONGING TO THE RIBONUCLEASE FAMILY<0}

(30) Priorité: 17.11.2006 FR 0654965; 12.12.2006 US 874257 P
(43) Date de publication de la demande: 19.08.2009
(73) Titulaire: L'Oréal, 75008 Paris (FR)
(72) Inventeur: DONOVAN, Mark, 95810 Berville (FR); BERNARD, Dominique, 92170 Vanves (FR); CASTIEL, Isabelle, 06200 Nice (FR); CHAUSSADE, Véronique, 75007 Paris (FR)
(74) Mandataire: Nony
(86) Numéro de dépôt international: PCT/FR2007/001887
(87) Numéro de publication internationale: WO 2008/068422

(56) Documents cités:
- EP-A- 0 943 679
- EP-A- 1 600 501
- WO-A-00/73452
- WO-A-2004/028479
- FR-A- 2 908 784
- STICHERLING M ET AL: "Abstract 104: Detection of RNase 7 immunoreactivity in inflammatory skin diseases." JOURNAL OF INVESTIGATIVE DERMATOLOGY, vol. 121, no. 1, juillet 2003 (2003-07), XP009087978 & INTERNATIONAL INVESTIGATIVE DERMATOLOGY 2003 : JOINT MEETING OF THE EUROPEAN SOCIETY FOR DERMATOLOGI; MIAMI BEACH, FLORIDA, USA; APRIL 30-MAY 04, 2003 ISSN: 0022-202X
- HARDER JUERGEN ET AL: "Psoriatic scales: a promising source for the isolation of human skin-derived antimicrobial proteins" JOURNAL OF LEUKOCYTE BIOLOGY, vol. 77, no. 4, avril 2005 (2005-04), pages 476-486, XP009087974 ISSN: 0741-5400
- HAISCH K ET AL: "Staphylococcus aureus strongly induces mRNA of the antimicrobial protein RNase 7 and proinflammatory cytokines in the human keratinocyte cell line HaCaT." ARCHIVES OF DERMATOLOGICAL RESEARCH, vol. 295, no. 8-9, février 2004 (2004-02), page 364, XP009045130 & 31ST ANNUAL MEETING OF THE ARBEITSGEMEINSCHAFT DERMATOLOGISCHE FORSCHUNG IN COOPERATION WITH THE DEU; BERLIN, GERMANY; FEBRUARY 26-28, 2004 ISSN: 0340-3696
- HARDER JUERGEN ET AL: "RNase 7, a novel innate immune defense antimicrobial protein of healthy human skin." JOURNAL OF BIOLOGICAL CHEMISTRY, vol. 277, no. 48, 29 novembre 2002 (2002-11-29), pages 46779-46784, XP009087965 ISSN: 0021-9258 cité dans la demande

1 SAKPKGMTSS QWFKI SEQUENCE LISTING
<110> L'OREAL
<120> Utilisation cosmétique d'une protéine de la famille des ribonucléases
<130> BR80830/CR/HG/klp
<140> 06 54965
   <141> 2006-11-17
<160> 7
<170> PatentIn version 3.1
<210> 1
   <211> 471
   <212> DNA
   <213> Homo Sapiens
<400> 1
   atggcaccgg ccagagcagg attctgcccc cttctgctgc ttctgctgct ggggctgtgg     60
   gtggcagaga tcccagtcag tgccaagccc aagggcatga cctcatcaca gtggtttaaa    120
   attcagcaca tgcagcccag ccctcaagca tgcaactcag ccatgaaaaa cattaacaag    180
   cacacaaaac ggtgcaaaga cctcaacacc ttcctgcacg agcctttctc cagtgtggcc    240
   gccacctgcc agacccccaa aatagcctgc aagaatggcg ataaaaactg ccaccagagc    300
   cacgggcccg tgtccctgac catgtgtaag ctcacctcag ggaagtatcc gaactgcagg    360
   tacaaagaga agcgacagaa caagtcttac gtagtggcct gtaagcctcc ccagaaaaag    420
   gactctcagc aattccacct ggttcctgta cacttggaca gagtccttta g             471
<210> 2
   <211> 387
   <212> DNA
   <213> Homo Sapiens
<400> 2
   aagcccaagg gcatgacctc atcacagtgg tttaaaattc agcacatgca gcccagccct     60
   caagcatgca actcagccat gaaaaacatt aacaagcaca caaaacggtg caaagacctc    120
   aacaccttcc tgcacgagcc tttctccagt gtggccgcca cctgccagac ccccaaaata    180
   gcctgcaaga atggcgataa aaactgccac cagagccacg ggcccgtgtc cctgaccatg    240
   tgtaagctca cctcagggaa gtatccgaac tgcaggtaca aagagaagcg acagaacaag    300
   tcttacgtag tggcctgtaa gcctccccag aaaaaggact ctcagcaatt ccacctggtt    360
   cctgtacact tggacagagt cctttag                                       387
<210> 3
   <211> 84
   <212> DNA
   <213> Homo Sapiens
<400> 3
   atggcaccgg ccagagcagg attctgcccc cttctgctgc ttctgctgct ggggctgtgg 60
   gtggcagaga tcccagtcag tgcc 84
<210> 4
   <211> 156
   <212> PRT
   <213> Homo Sapiens
<400> 4
   Met Ala Pro Ala Arg Ala Gly Phe Cys Pro Leu Leu Leu Leu Leu Leu
   1 5 10 15
   Leu Gly Leu Trp Val Ala Glu Ile Pro Val Ser Ala Lys Pro Lys Gly
   20 25 30
   Met Thr Ser Ser Gln Trp Phe Lys Ile Gln His Met Gln Pro Ser Pro
   35 40 45
   Gln Ala Cys Asn Ser Ala Met Lys Asn Ile Asn Lys His Thr Lys Arg
   50 55 60
   Cys Lys Asp Leu Asn Thr Phe Leu His Glu Pro Phe Ser Ser Val Ala
   65 70 75 80
   Ala Thr Cys Gln Thr Pro Lys Ile Ala Cys Lys Asn Gly Asp Lys Asn
   85 90 95
   Cys His Gln Ser His Gly Pro Val Ser Leu Thr Met Cys Lys Leu Thr
   100 105 110
   Ser Gly Lys Tyr Pro Asn Cys Arg Tyr Lys Glu Lys Arg Gln Asn Lys
   115 120 125
   Ser Tyr Val Val Ala Cys Lys Pro Pro Gln Lys Lys Asp Ser Gln Gln
   130 135 140
   Phe His Leu Val Pro Val His Leu Asp Arg Val Leu
   145 150 155
<210> 5
   <211> 128
   <212> PRT
   <213> Homo Sapiens
<400> 5
   Lys Pro Lys Gly Met Thr Ser Ser Gln Trp Phe Lys Ile Gln His Met
   1 5 10 15
   Gln Pro Ser Pro Gln Ala Cys Asn Ser Ala Met Lys Asn Ile Asn Lys
   20 25 30
   His Thr Lys Arg Cys Lys Asp Leu Asn Thr Phe Leu His Glu Pro Phe
   35 40 45
   Ser Ser Val Ala Ala Thr Cys Gln Thr Pro Lys Ile Ala Cys Lys Asn
   50 55 60
   Gly Asp Lys Asn Cys His Gln Ser His Gly Pro Val Ser Leu Thr Met
   65 70 75 80
   Cys Lys Leu Thr Ser Gly Lys Tyr Pro Asn Cys Arg Tyr Lys Glu Lys
   85 90 95
   Arg Gln Asn Lys Ser Tyr Val Val Ala Cys Lys Pro Pro Gln Lys Lys
   100 105 110
   Asp Ser Gln Gln Phe His Leu Val Pro Val His Leu Asp Arg Val Leu
   115 120 125
<210> 6
   <211> 28
   <212> PRT
   <213> Homo Sapiens
<400> 6
   Met Ala Pro Ala Arg Ala Gly Phe Cys Pro Leu Leu Leu Leu Leu Leu
   1 5 10 15
   Leu Gly Leu Trp Val Ala Glu Ile Pro Val Ser Ala
   20 25
<210> 7
   <211> 15
   <212> PRT
   <213> Homo Sapiens
<400> 7
   Ser Ala Lys Pro Lys Gly Met Thr Ser Ser Gln Trp Phe Lys Ile
   1 5 10 15

## Description

La présente invention a pour objet l'utilisation, en cosmétique et en thérapeutique, d'au moins une protéine antimicrobienne de la famille des ribonucléases, la ribonucléase 7, ou de polypeptides dérivés de cette protéine, à titre de marqueur pour l'évaluation d'un état de l'épiderme d'une peau sèche.

Parmi ses nombreuses fonctions physiologiques, l'épiderme possède celle de constituer une barrière à la fois biologique, physique et chimique contre l'invasion de l'organisme par les microorganismes.

Les propriétés de barrière physique de l'épiderme sont, notamment, liées à son organisation structurelle.

Ainsi l'épiderme est conventionnellement divisé en une couche basale de kératinocytes constituant la couche germinative de l'épiderme, une couche dite épineuse constituée de plusieurs couches de cellules polyédriques et enfin, un ensemble de couches supérieures appelées couches cornées (ou *stratum corneum*), constituées de kératinocytes au stade terminal de leur différenciation appelés cornéocytes.

Les propriétés de barrière chimique de l'épiderme dépendent, notamment, de la libération à la surface de ce dernier de nombreuses protéines antimicrobiennes telles que des cathélicidines, l'antileukoprotéase inhibitrice de sérine protéase, la dermcidine ou les protéines de la famille des β-défensines.

De nombreux troubles ou pathologies cutanés ou du cuir chevelu peuvent résulter d'un dysfonctionnement de l'organisation structurelle cellulaire épidermique, ou d'un défaut de la fonction barrière chimique de l'épiderme, comme la sécheresse cutanée, le psoriasis, les dermatites atopiques, l'ichtyose, l'hyperkératose, des réactions inflammatoires, issues d'infections microbiennes, ou des états pelliculaires.

A ce jour, on ne dispose que de peu de marqueurs biologiques permettant de déterminer efficacement et précisément l'état d'un épiderme, et notamment de relier un trouble cutané ou du cuir chevelu à un dysfonctionnement de ses fonctions physiologiques.

Sticherling et al. (J. Invest. Dermatol., 121, 2003), Harder & Schroder (J. Leukoc. Biol., 77, 2005 : 476) et la demande WO 2004/028479 décrivent l'identification de certaines protéines dans des peaux issues de patients atteints de psoriasis.

EP 0943679 et Haisch et al. (Arch. Dermatol. Res., 2004, 295 : 364) enseignent l'expression de protéines anti-microbiennes dans les kératinocytes.

WO 00/73452 enseigne l'utilisation d'une sélection de protéines pour le traitement de maladies associées au système immunitaire et à l'inflammation, lesquelles peuvent inclure des maladies de la peau.

Ainsi, il existe un besoin de disposer de marqueurs biologiques permettant de caractériser précisément un état d'un épiderme.

Il existe également un besoin de disposer d'un marqueur biologique qui puisse être utilisé de manière fiable dans un procédé de diagnostic d'un trouble cutané ou du cuir chevelu.

Il existe également un besoin d'identification, dans l'épiderme, de nouvelles cibles cosmétiques ou thérapeutiques.

Il existe également un besoin de disposer de nouveaux outils de criblage de molécules susceptibles d'exercer une action cosmétique ou thérapeutique sur l'épiderme ou les matières kératiniques en général. Au sens de l'invention, on entend désigner par "épiderme" aussi bien la peau que le cuir chevelu.

Il existe également un besoin de disposer de nouvelles compositions cosmétiques ou thérapeutiques utilisables pour le traitement de troubles cutanés ou du cuir chevelu résultant des dysfonctionnements précités.

La présente invention a pour objet de satisfaire à l'ensemble de ces besoins.

Au sens de la présente invention, on entend par les expressions "agent modulateur" ou "composé chimique ou biologique, susceptible ou apte à moduler l'activité biologique et/ou l'expression et/ou l'interaction avec un élément d'une matrice extracellulaire" tout composé susceptible ou apte à agir, directement ou indirectement, sur au moins un polypeptide conforme à l'invention, une séquence d'acides nucléiques codant pour celui-ci, sur un élément d'une voie de signalisation intra ou extra-cellulaire ou d'une voie métabolique impliquant ledit polypeptide, sur un élément de matrice extracellulaire, ou sur un élément impliqué dans la régulation de la transcription et/ou la traduction d'une séquence d'acides nucléiques codant pour ledit polypeptide.

De tels agents modulateurs sont plus particulièrement décrits ci-après.

Selon un de ses aspects, la présente invention se rapporte à un procédé *in vitro* ou *ex vivo* d'évaluation d'un état d'un épiderme d'une peau sèche comprenant au moins les étapes consistant à :
a) déterminer une teneur d'un polypeptide de séquence d'acides aminés codée par une séquence d'acides nucléiques représentée par une séquence choisie parmi SEQ ID NO 1, SEQ ID NO 2, ou SEQ ID NO 3, un analogue de celles-ci présentant une homologie de séquence d'au moins 85% et une activité biologique de même nature, ou d'acides nucléiques codant pour ledit polypeptide, dans un échantillon d'épiderme, et
b) comparer ladite teneur déterminée à l'étape a) à une valeur de référence, l'état de l'épiderme étant caractérisé par une détermination de la teneur déterminée à l'étape a) comme étant de 5 à 10 fois inférieure à la valeur de référence.

Le procédé peut être effectué *in vitro* ou *ex vivo.*

Au sens de l'invention, le terme « cosmétique » doit se comprendre selon la définition donnée par la Directive 76/768/CEE, et en particulier vise à désigner des compositions ou traitements destinés à être mis en oeuvre sur des matières kératiniques, par exemple la peau, les muqueuses, les poils et les cheveux, en vue de les nettoyer, de les parfumer, d'en modifier l'aspect, notamment esthétique, et/ou de les protéger ou de les maintenir en bon état.

Ainsi, la présente invention repose plus particulièrement sur l'identification par les inventeurs, de la ribonucléase 7 à titre d'agent et/ou de cible cosmétique ou pharmaceutique au niveau du *stratum corneum* de l'épiderme, ainsi qu'au niveau du follicule pileux.

En particulier, cette identification résulte de l'observation inattendue selon laquelle la RNase 7 est différentiellement exprimée dans le *stratum corneum* d'un épiderme normal et jeune et d'un épiderme sec, en particulier un épiderme âgé.

Ainsi, comme il ressort des exemples présentés ci-après, les inventeurs ont déterminé la teneur en protéine RNase 7, dont la séquence d'acides aminés est représentée par la séquence SEQ ID NO 5, dans l'épiderme d'individus jeunes et d'individus âgés, et notamment de femmes jeunes non ménopausées et de femmes âgées ménopausées. Ils ont observé, de manière surprenante, que la teneur de cette protéine était plus élevée dans l'épiderme des femmes jeunes que dans l'épiderme des femmes âgées, et était également plus élevée dans les épidermes normalement hydratés que dans les épidermes secs.

Il est connu qu'une augmentation de la sécheresse cutanée est souvent observée avec l'âge, toutefois de tels états de sécheresse cutanée peuvent également se manifester chez les sujets jeunes. En effet, l'état de sécheresse cutanée est un état physiologique qui peut être présent chez des sujets jeunes, sans aucune cause pathologique, du moins apparente. L'origine de cette sécheresse peut être constitutionnelle ou acquise. Ainsi, de nombreux facteurs extérieurs peuvent entraîner l'assèchement de la peau ou aggraver l'état d'une peau déjà sèche. Parmi ces facteurs on peut citer le conditions climatiques difficiles, les rayons solaires, l'exposition à certains agents chimiques ou thérapeutiques (par exemple les rétinoïdes). Cette sécheresse se manifeste généralement par une sensation de tiraillements et/ou de tension. Celle-ci est rugueuse au toucher et apparaît couverte de squames.

De manière inattendue, les inventeurs ont donc constaté que la ribonucléase 7 constitue une cible pour agir efficacement sur ces troubles cutanés.

La ribonucléase 7 (RNase 7) est une endonucléase appartenant à la famille des ribonucléases et, notamment, de la super-famille A, comptant actuellement treize membres (Cho et al., Genomics, 2005, 85:208-220).

Les ribonucléases sont des enzymes hydrolytiques capables de couper des liaisons nucléotidiques.

La RNase 7 présente en particulier une activité anti-microbienne.

La ribonucléase 7 a été identifiée et localisée par HARDER & SCHROEDER (J. Biol. Chem., 2002, 277 : 46779-46784) dans le *stratum corneum* de l'épiderme et à partir de cultures primaires de kératinocytes. Ces derniers ont également montré que cette protéine était dotée de propriétés antimicrobiennes.

La RNase 7 est synthétisée sous la forme d'une proprotéine de 156 acides aminés (SEQ NO 4) contenant un site catalytique de type ribonucléase pancréatique et un peptide signal de 28 acides aminés à l'extrémité N-terminale (SEQ NO 6). L'élimination du peptide signal conduit à la génération d'une enzyme active de 128 acides aminés (protéine SEQ NO 5).

Au sens de la présente invention, on entend désigner de manière générale, sauf indications contraires, par RNase 7, la forme proprotéine (SEQ ID NO 4), et la forme protéine (SEQ ID NO 5).

### POLYPEPTIDE SELON L'INVENTION

Selon un mode de réalisation, un polypeptide convenant à l'invention peut avoir une séquence d'acides aminés codée par une séquence d'acides nucléiques représentée en tout ou partie par une séquence choisie parmi SEQ ID NO 1, SEQ ID NO 2, ou SEQ ID NO 3, un analogue de celle-ci présentant une homologie de séquence d'au moins 85% et une activité biologique de même nature.

Selon un mode de réalisation particulier, un polypeptide convenant à l'invention peut avoir une séquence d'acides aminés représentée en tout ou partie par une séquence choisie parmi SEQ ID NO 4, SEQ ID NO 5, SEQ ID NO 6, ou SEQ ID NO 7, ou un analogue de celle-ci présentant une homologie de séquence d'au moins 85% et une activité biologique de même nature.

Par "analogue d'un polypeptide", on entend désigner tout polypeptide présentant une homologie de séquence, en particulier vis-à-vis d'une des séquences caractéristiques dudit polypeptide, ainsi qu'une activité biologique de même nature, portée par exemple par des séquences peptidomimétiques.

L'homologie peut être d'au moins 85 %, par exemple d'au moins 90 %, et par exemple d'au moins 95 %. L'homologie peut être déterminée par comparaison visuelle ou tout moyen informatique généralement utilisé dans le domaine.

L'homologie de séquence peut résulter de modifications issues de mutations ou de variations dans les séquences des peptides selon l'invention provenant soit de la délétion d'un ou plusieurs acides aminés, ou de l'insertion d'un ou plusieurs acides aminés, ou encore de la substitution d'un ou plusieurs acides aminés dans les séquences caractéristiques des polypeptides selon l'invention.

Par "séquence caractéristique du polypeptide", on entend viser notamment au regard de la RNase 7, la proprotéine (SEQ ID NO 4), la protéine active (SEQ ID NO 5), le peptide signal (SEQ ID NO 6), le site actif comprenant les acides aminés en position 43 et 151, et comprenant le cas échéant les acides aminés entourant ces acides aminés, à raison, par exemple, de 1, voire de 2, voire de 3, voire de 4, voire de 5, voire de 6, voire de 7, voire de 8 acides aminés de part et d'autre du site actif, les sites de liaison du substrat représenté par la séquence d'acides aminés 66 à 70 et l'acide aminé en position 91, et comprenant le cas échéant les acides aminés de part et d'autre de ce site de liaison, à raison, par exemple, de 1, voire de 2, voire de 4, voire de 8 acides aminés de part et d'autre de ces sites, ainsi que la séquence SEQ ID NO 7, représentant un épitope dérivé de la RNase 7.

De manière générale, les analogues polypeptidiques peuvent comprendre des substitutions conservatrices par rapport à la séquence d'acides aminés du polypeptide naturel.

Plusieurs de ces modifications peuvent être combinées.

A titre d'exemple de mutations que l'on peut considérer dans la présente invention, il peut être mentionné le remplacement d'un ou plusieurs résidus d'acides aminés par des résidus d'acides aminés ayant un indice hydropathique similaire sans pour autant affecter de manière sensible les propriétés biologiques du polypeptide, et notamment son activité biologique telle que son activité ribonucléase.

L'indice hydropathique est un indice attribué aux acides aminés en fonction de leur hydrophobicité et de leur charge (Kyte et Doolittle (1982), J. Mol. Biol., 157 : 105).

Un polypeptide ou analogue également visé par la présente invention peut être un polypeptide ayant subi une ou plusieurs modification(s) post-traductionnelle(s).

Par "modification(s) post-traductionnelle(s)", on entend englober l'ensemble des modifications qu'un peptide ou une protéine est susceptible de subir à l'issu de sa synthèse dans une cellule, telle que par exemple une ou des phosphorylation(s), une ou des glycosylation(s), une ou des lipidation(s), telles qu'une farnésylation ou une palmitoylation, un réarrangement structural de type formation de ponts disulfures et/ou clivage à l'intérieur de la séquence peptidique.

L'analogue peut, par ailleurs, présenter sensiblement la même activité biologique que le polypeptide naturel.

Selon un mode de réalisation, un polypeptide convenant à la mise en oeuvre de l'invention peut également être un polypeptide naturel ou synthétique, le cas échéant susceptible d'être obtenu après protéolyse de la RNase 7 ou par synthèse chimique ou biologique ou par extraction à partir d'un tissu biologique, comme par exemple la peau, exprimant naturellement ou après transduction ce polypeptide, ainsi que les différentes formes post-traductionnelles de celui-ci, ou encore tout polypeptide naturel ou synthétique dont la séquence comprend totalement ou partiellement (en tout ou partie) une séquence d'acides aminés précitée, par exemple les variants et les analogues.

Selon un autre mode de réalisation, un polypeptide convenant à la mise en oeuvre de l'invention peut également être un polypeptide tel que défini précédemment fusionné avec un autre polypeptide, un agent de ciblage hydrophile ou hydrophobe, un précurseur de bio-conversion, un agent de marquage luminescent, radioactif ou colorimétrique.

De manière non limitative, on peut citer comme exemple de composés susceptibles d'être couplés avec un polypeptide conforme à l'invention des protéines fluorescentes comme la "Green Fluorescent Protein", des composés chimiques fluorescents tels que la rhodamine, la fluorescéine, ou le Texas Red, des composés phosphorescents, des éléments radioactifs, tels que ³H, ³⁵5, ¹²¹I ou ¹²⁵I, ou des agents de marquage colorimétriques comme les substrats chromogènes sensibles à l'action de la galactosidase, de la peroxydase, de la chloramphénicol acétyltransférase, de la luciférase ou de la phosphatase alcaline.

Selon la nature des composés susceptibles d'être couplés avec un polypeptide conforme à l'invention, le couplage peut être opéré par des procédés chimiques, notamment au moyen de fonctions chimiques réactives ou par des procédés de biologie moléculaire connus de l'homme de l'art.

### ACIDE NUCLEIQUE SELON L'INVENTION

Au sens de la présente invention, on entend désigner par "fragment de séquence d'acides nucléiques", une séquence d'acides nucléiques codant pour un polypeptide conforme à l'invention, ou un analogue de celui-ci présentant une homologie de séquence d'au moins 85% et une activité biologique de même nature, et en particulier une séquence d'acides nucléiques choisie parmi SEQ ID NO 1 (codant pour la forme proprotéine), SEQ ID NO 2 (codant pour la forme protéine active), SEQ ID NO 3 (codant pour le peptide signal), un analogue de celle-ci, ou un fragment de celle-ci.

Par "analogue d'une séquence d'acides nucléiques", on entend désigner toute séquence d'acides nucléiques, éventuellement résultant de la dégénérescence du code des acides nucléiques, et codant pour une séquence d'un polypeptide identique ou analogue à la séquence du polypeptide codée par ladite séquence d'acides nucléiques.

Une séquence d'acides nucléiques conforme à l'invention peut être une séquence sens, anti-sens ou interférentielle correspondant à une séquence codant pour un polypeptide conforme à l'invention.

Les séquences d'acides nucléiques selon l'invention peuvent notamment être utilisées pour préparer des séquences d'acides ribonucléiques correspondantes, sens ou antisens.

Une séquence d'acides nucléiques convenant à l'invention peut notamment être telle que définie précédemment.

Selon un mode de réalisation, une séquence d'acides nucléiques peut représenter une séquence d'acides nucléiques sens, anti-sens ou interférentielle correspondant à ladite séquence d'acides nucléiques codant pour un polypeptide conforme à l'invention.

Les séquences d'acides nucléiques peuvent être issues de toutes origines possibles à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

### PROCEDE D'EVALUATION DE L'ETAT D'UN EPIDERME SELON L'INVENTION

Selon un mode de réalisation, la présente invention concerne un procédé *in vitro* ou *ex vivo* d'évaluation d'un état d'un épiderme d'une peau sèche comprenant au moins les étapes consistant à :
a) déterminer, dans un échantillon d'épiderme, une teneur d'un polypeptide de séquence d'acides aminés codée par une séquence d'acides nucléiques représentée par une séquence choisie parmi SEQ ID NO 1, SEQ ID NO 2, ou SEQ ID NO 3, un analogue de celles-ci présentant une homologie de séquence d'au moins 85% et une activité biologique de même nature, ou d'acides nucléiques codant pour ledit polypeptide, et
b) comparer ladite teneur déterminée à l'étape a) à une valeur de référence, l'état de l'épiderme étant caractérisé par une détermination de la teneur déterminée à l'étape a) comme étant de 5 à 10 fois inférieure à la valeur de référence.

Un procédé selon l'invention peut être effectué *in vitro* ou *ex vivo.*

Selon un mode de réalisation, un procédé selon l'invention peut être effectué sur un échantillon d'épiderme prélevé chez un individu.

Un procédé selon l'invention peut également être effectué sur un échantillon d'épiderme prélevé sur une peau isolée reconstruite afin d'en qualifier l'état.

La détermination d'un état d'un épiderme peut être indicatif d'un éventuel trouble cutané ou du cuir chevelu. En particulier un état de l'épiderme peut être indicatif d'un éventuel trouble cutané, notamment choisi parmi une sécheresse excessive de l'épiderme, une xérose, une ichtyose, une atopie, une dermatite atopique, un psoriasis, une hyperkératose, un eczéma, une rosacée, un lichen, un prurit, une couperose, une dermite séborrhéique, une kératodermie palmo-plantaire et un prurigos.

Un procédé selon l'invention peut également être effectué sur un cuir chevelu.

### - Modes de prélèvement de l'échantillon

Le prélèvement d'un échantillon d'épiderme peut se faire par toute méthode connue de l'homme de l'art.

Ces méthodes peuvent être effectuées par des techniques dites de stripping.

Ces strippings sont des surfaces collantes appliquées à la surface de l'épiderme comme le blenderm de 3M, le D'squam (adhésif commercial de CuDERM), la colle cyanoacrylate ou la méthode du stripping vernis. Grâce à ces strippings, les cornéocytes adhérents et le contenu de leurs espaces intercellulaires peuvent être prélevés et soumis par la suite à une extraction permettant d'avoir accès au contenu protéique.

Le prélèvement d'un échantillon convenant au procédé peut aussi être effectué de façon plus directe par "lavages" de la surface cutanée au moyen par exemple d'accessoires de type turbine à ailette, de type cellule à spirale (tel que décrit dans le brevet FR 2 667 778) associée à un circuit fluidique, ou simplement par ajout/prélèvement d'une goutte de tampon à la surface de la peau.

A titre indicatif, d'autres méthodes de prélèvement adaptées à la mise en oeuvre de l'invention, on peut mentionner des méthodes par grattage de la partie supérieure du *stratum corneum* au moyen d'un système bilames. Cette technique permet de recueillir des squames qui peuvent ensuite être directement analysées par différentes techniques pour déterminer les taux en minéraux, aminoacides ou lipides. Les prélèvements peuvent être conservés au congélateur et analysés ensuite par des techniques connues de l'homme du métier, telles que l'analyse LC/ESI/MS.

### - Valeur de référence

Une valeur de référence peut être, par exemple, une teneur en polypeptide ou en séquence d'acides nucléiques déterminée sur un échantillon d'épiderme prélevé sur une peau présentant une hydratation normale, et en particulier sur une peau jeune. Dans le cadre de la présente invention, il a été observé que le taux de polypeptide de type RNase 7 peut diminuer de façon physiologique chez les peaux de sujets âgés de plus de 45 ans et en particulier chez les femmes.

On entend donc par peau jeune, une peau d'un sujet âgé de moins de 45 ans environ, par opposition à une peau mature.

Les valeurs de RNase 7 peuvent être exprimées en unités arbitraires ou être calculées grâce à l'établissement d'une courbe d'étalonnage effectuée grâce à une protéine recombinante ou purifiée à partir d'un épiderme. Dans tous les cas, le taux de RNase 7 mesuré peut être exprimé par rapport à la quantité totale de protéines présentes dans l'extrait. La quantité totale de protéines peut être mesurée par exemple par la méthode de Bradford.

La mesure d'une valeur de référence peut être effectuée parallèlement ou séquentiellement à la détermination de ladite teneur d'un polypeptide ou d'une séquence d'acides nucléiques.

Une comparaison d'une teneur déterminée avec une valeur de référence peut permettre d'évaluer une déviation par rapport à cette valeur.

L'analyse de l'intensité et/ou de la nature de cette déviation (négative ou positive) peut être informative de l'état de l'épiderme.

Ainsi, par exemple, une teneur en polypeptide déterminée de 5 à 10 fois inférieure à une valeur de référence obtenue à partir d'une peau normale peut être indicatif d'un trouble cutané. De manière conventionnelle, une peau normale qualifie une peau saine qui n'est ni sèche, ni grasse.

Plus particulièrement, les troubles cutanés ou du cuir chevelu susceptibles d'être identifiés par mise en oeuvre d'un procédé d'évaluation selon l'invention, peuvent être ceux résultant d'une déficience ou d'une surexpression de la protéine RNase 7, comme par exemple ceux indiqués ci-après.

### - Modes de détermination du polypeptide

Un polypeptide convenant à la mise en oeuvre d'un procédé selon l'invention peut être avantageusement la protéine RNase 7.

La détermination d'une teneur en polypeptide conforme à l'invention ou en acides nucléiques conformes à l'invention dans un échantillon d'épiderme peut être effectuée par tout protocole connu de l'homme de l'art.

A titre de méthode de détection d'un polypeptide, il peut être fait mention du Western blot, du Slot blot, du Dot blot, des méthodes ELISA (Enzyme linked immuno-Sorbent Assay) de type singleplex ou multiplex, des méthodes de protéomiques, de coloration de polypeptides dans un gel de polyacrylamide par un colorant à base d'argent, par le bleu de Coomassie ou par le SYPRO, de l'immunofluorescence, de l'absorption UV, de méthodes immunohistochimiques en microscopie classique, électronique ou confocale, de FRET (fluorescence résonance energy transfer/transfert d'énergie par résonance de fluorescence), des méthodes de TR-FRET (time resolved FRET/FRET en résolution de temps), des méthodes de FLIM (fluorescence lifetime imaging microscopy/imagerie par microscopie en temps de fluorescence), des méthodes de FSPIM (fluorescence spectral imaging microscopy/imagerie par microscopie en spectre de fluorescence), des méthodes de FRAP (fluorescence recovery after photobleaching/recouvrement de fluorescence après photo blanchiment), des méthodes par gène reporteur, des méthodes d'AFM (atomic force microscopy/microscopie par force atomique), des méthodes par résonance plasmonique de surface, des méthodes de microcalorimétrie, des méthodes de cytométrie en flux, des méthodes de biosenseurs, des méthodes de radioimmunoessais (RIA), des méthodes mettant en oeuvre des puces à peptides, des puces à sucre, des puces d'anticorps, des méthodes de spectrométrie de masse, des méthodes de spectrométrie de type SELDI-TOF (Ciphergen).

De façon plus générale, des méthodes de dosage immunoenzymatiques à partir de solutions de protéines, plus quantitatives et sensibles peuvent en particulier être utilisées. Ces méthodes de type ELISA associent des couples d'anticorps de capture et de détection spécifiques de l'antigène ciblé. Des anticorps commerciaux ou des anticorps polyclonaux, monoclonaux ou recombinants développés spécifiquement peuvent être utilisés. Des techniques ELISA multiplexes de grande capacité peuvent également être mises en oeuvre. On peut ainsi citer l'approche multiplexe de type anticorps sur billes Luminex (par exemple Bioplex de Bio-Rad), de type anticorps sur surface plane ("antibodies-arrays") (par exemple approche proposée par la société Meso scale Discovery).

En particulier, il peut être avantageux de détecter la présence d'un polypeptide conforme à l'invention au moyen d'un anticorps, le cas échéant sous une forme marquée. Un tel anticorps peut être marqué au moyen d'une substance détectable directement ou détectable par réaction avec un autre réactif.

Par "anticorps", on entend désigner de manière générale des anticorps monoclonaux ou polyclonaux, ainsi que des fragments d'immunoglobuline susceptibles de lier un antigène et qui peuvent être produits par toute technique de génie génétique connue de l'homme de l'art ou par coupure enzymatique ou chimique d'anticorps intact.

Ainsi, selon un de ses aspects, la présente invention concerne également l'utilisation d'un anticorps se liant spécifiquement à un polypeptide conforme à l'invention à titre d'outil d'évaluation d'un état d'un épiderme.

Selon un mode de réalisation, un anticorps convenant à l'invention peut se lier spécifiquement à un polypeptide représenté par la séquence SEQ ID NO 4, ou des épitopes présents dans cette séquence, ou des analogues de ceux-ci, notamment à un épitope de séquence d'acides aminés représentée par la séquence SEQ ID NO 7, ou un analogue de celle-ci.

Un anticorps susceptible d'être utilisé à titre d'outil d'évaluation d'un état d'un épiderme peut être obtenu par tout procédé connu de l'homme de l'art, tel que décrit dans "Antibodies: A Laboratory Manual", Cold Spring Harbor Laboratory Press, Cold Spring Harbor, N.Y. (1990).

Ainsi, selon un de ses aspects, la présente invention concerne également l'utilisation d'un anticorps recombinant de type monovalent ou divalent qui peut être obtenu par un procédé bactériophage display tel que proposé par la société Antibodies By Design.

### - Modes de détermination de l'acide nucléique

Une séquence d'acides nucléiques convenant à la mise en oeuvre d'un procédé selon l'invention peut être avantageusement une séquence d'acides nucléiques codant pour la RNase 7, par exemple de type ARNm.

A titre d'exemple de méthodes de détection de séquence d'acides nucléiques, il peut être fait mention de la réaction de polymérisation en chaîne (PCR), de la réaction de polymérisation en chaîne transcriptase inverse (RT-PCR), de la réaction de polymérisation en chaîne quantitative ou en temps réel (Q-PCR), du Northern blot, du RPA (ribonuclease protection assay), des puces à ADN, des puces à oligonucléotides, des puces transcriptomiques, de méthodes d'hybridation *in situ*.

A titre d'exemple d'agents convenant à la détection d'une séquence d'acides nucléiques, et en particulier d'ARNm, il peut être fait mention de sonde d'acides nucléiques marquée pouvant s'hybrider à ladite séquence.

De telles sondes d'acides nucléiques peuvent être aisément obtenues par toute méthode connue de l'homme de l'art.

Ainsi, les séquences d'acides nucléiques conformes à l'invention peuvent être utilisées pour réaliser des amorces oligonucléotidiques sens et/ou antisens, qui s'hybrident à la séquence SEQ ID NO : 1, SEQ ID NO : 2, ou SEQ ID NO : 3, ou un fragment de celle-ci ou un analogue de celle-ci.

Avantageusement, l'activité biologique dont la valeur peut être déterminée peut être une activité enzymatique de type ribonucléase.

Toute méthode de mesure d'une activité enzymatique connue de l'homme de l'art peut être mise en oeuvre dans un procédé conforme à l'invention.

Par exemple, il peut être utilisé une méthode mettant en oeuvre une mesure de l'hydrolyse d'un substrat fluorescent, spécifique pour une ribonucléase en utilisant une préparation de RNase 7 purifiée à partir de *stratum corneum* (SC). La mesure de l'hydrolyse dudit substrat fluorescent est soit de type "point final" ou de type "cinétique" et l'activité enzymatique peut être rapportée par quantité de protéines totales des échantillons.

Par "activité biologique", on entend désigner notamment au regard de la RNase 7, l'activité biologique de la pro-protéine (SEQ ID NO 4), du peptide signal (SEQ ID NO 6), l'activité biologique de la protéine RNase 7 se traduisant par une activité endonucléasique (SEQ ID NO 5) ainsi que les propriétés immunogènes d'un épitope identifié par la séquence SEQ ID NO 7.

Selon un mode de réalisation, l'activité biologique du polypeptide peut être comparée à une valeur de référence.

Une valeur de référence peut être obtenue en effectuant un procédé conforme à l'invention en l'absence de tout composé biologique ou chimique à tester.

Selon un autre mode de réalisation, la détermination de la propriété d'un composé chimique ou biologique à moduler l'activité biologique d'un polypeptide conforme à l'invention peut également être déterminée par détermination de la capacité dudit composé à moduler la liaison dudit polypeptide à un substrat.

Avantageusement, un polypeptide mis en oeuvre dans un procédé selon la présente invention peut être la protéine RNase 7.

L'expression d'une séquence d'acides nucléiques peut être déterminée, par exemple, au moyen de sondes oligonucléotidiques, par des protocoles connus de l'homme de l'art.

L'expression d'une séquence d'acides nucléiques conforme à l'invention peut être comparée à une valeur de référence obtenue, par exemple, en effectuant un procédé conforme à l'invention en l'absence de composé à tester.

Au sens de l'invention, on entend par "prévention d'un trouble", la réduction du risque de survenue du trouble considéré, notamment tel que défini précédemment.

Au sens de la présente invention, on entend désigner par l'expression "quantité efficace" la quantité minimale nécessaire à l'observation de l'effet attendu, à savoir un effet cosmétique ou un effet thérapeutique, étant entendu que les quantités efficaces nécessaires à l'obtention d'un effet cosmétique ou d'un effet thérapeutique peuvent être, le cas échéant, identiques ou différentes.

Au sens de la présente invention, on entend désigner par "milieu physiologiquement acceptable", un milieu convenant à l'application d'une composition sur une matière kératinique, telle que la peau, le cuir chevelu, les lèvres, les muqueuses et les fibres kératiniques comme les cheveux, les ongles et les poils.

Au sens de la présente invention, on entend désigner par "thérapeutique" une composition pouvant être mise en oeuvre dans le cadre d'un traitement prophylactique et/ou curatif, ou d'une méthode de diagnostic, d'un trouble cutané ou du cuir chevelu.

Les troubles cutanés plus particulièrement visés par la présente invention peuvent être la peau sèche, la peau très sèche, et chez les individus dont la peau présente un aspect âgé, notamment chez les femmes au-delà de 45 ans et/ou ménopausées, voire très âgées, les polypeptides, les séquences nucléiques, les agents modulateurs et les compositions conformes à l'invention peuvent être par ailleurs adaptées au traitement des xéroses et des peaux abîmées par les UV.

Au sens de la présente invention, "un" doit se comprendre, sauf indication contraire, au sens de "au moins un".

Les exemples figurant ci-après sont présentés à titre illustratif et non limitatif de l'invention.

### FIGURE

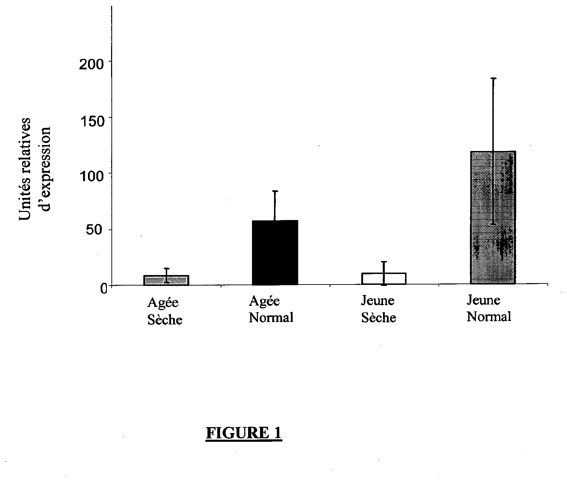

**Figure 1** **:** illustre un histogramme montrant l'expression moyenne de la RNase 7 dans 4 types distincts d'épidermes (colonne grise gauche : peau sèche âgée ; colonne noire : peau âgée normale ; colonne blanche : peau jeune sèche ; colonne grise droite : peau jeune normale).

L'expression en unités relatives représente l'intensité de la bande identifiée sur le WESTERN blot comme correspondant à la RNase 7 par rapport aux contrôles internes.

### EXEMPLE

### Exemple I- Evaluation de l'expression de la ribonucléase 7 dans l'épiderme

Des femmes volontaires en bonne santé avec une peau normale ou une peau sèche ont été recrutées pour l'étude et divisées en deux groupes, en fonction de l'âge : âge dit post-ménopause (55-60 ans) et âge dit pré-ménopause (35-40 ans).

Ainsi, quatre groupes ont été obtenus : femmes d'âge dit post-ménopause avec une peau normale (n=13), femmes d'âge dit post-ménopause avec une peau sèche (n=15), femmes d'âge dit pré-ménopause avec une peau normale (n=14) et femmes d'âge dit pré-ménopause avec une peau sèche (n=16).

Des échantillons du *stratum corneum* ont été prélevés au moyen de la technique dite du "stripping vernis" chez chacun des individus en utilisant un protocole modifié de LUNDSTROM et EGELRUD (Acta Derm Venereol, 1991, 71:471).

Les "stripping vernis" ont été lavés avec de l'acétone refroidie sur glace et les cornéocytes ont été récupérés après filtration et centrifugation.

Les protéines solubles ont été extraites dans un tampon PBS-0,1 % TX-100.

Chaque échantillon a été ajusté à la concentration de 0,15 mg/ml avant analyse par WESTERN blot.

10 µg de protéine ont été séparées sur un gel 4-20 % SDS-NuPage (InVitrogen).

Les protéines séparées ont ensuite été transférées sur une membrane PDVF (Millipore) en utilisant un système X-cell Blot (InVitrogen) selon les instructions du fabriquant.

La membrane a été ensuite saturée avec 1 % de lait écrémé dans du TBS-0,05 % Tween (TBS-T) pendant 1 heure à température ambiante.

La membrane a ensuite été incubée avec un anticorps polyclonal anti-RNase 7 à la dilution de 1:1000 pendant la nuit à 4 °C.

L'anticorps polyclonal anti-RNase 7 a été obtenu par immunisation de lapins avec la séquence peptidique N-terminale SEQ ID NO 7 de la protéine RNase 7.

L'anticorps obtenu a été purifié avec le même peptide que celui utilisé pour l'immunisation des lapins.

La membrane a ensuite été lavée avec le tampon TBS-T puis incubée en présence d'un anticorps secondaire anti-lapin couplé à la peroxydase pendant 1 heure à température ambiante à la dilution de 1:4000.

La membrane a ensuite été lavée comme décrit précédemment.

La détection par chemiluminescence a été réalisée avec ECLplus (GE Healthcare) selon les instructions du fabriquant.

Les images ont été acquises sur une période d'une minute par un Multi-imager Fluor-S max (BIORAD).

L'intensité de chaque bande a été quantifiée en utilisant Quantity One (BIORAD).

Les données ont été normalisées par rapport à un contrôle protéique interne marqué amido-black sur la même membrane et calculées sous la forme d'un rapport entre l'intensité de la bande de la protéine d'intérêt et l'intensité de la bande du contrôle interne.

Les résultats représentés en Figure 1 indiquent que l'expression de la protéine RNase 7 est fortement diminuée dans le stratum corneum des peaux sèches et également chez les femmes d'âge dit post-ménopause.

### LISTE DE SEQUENCES

**SEQ ID NO 1**
**SEO ID NO 2**
**SEQ ID NO 3**
**SEQ ID NO 4**
**SEQ ID NO 5**
**SEQ ID NO 6**
1 MAPARAGFCP LLLLLLLGLW VAEIPVSA
**SEO ID NO 7**
## Revendications

1. Procédé *in vitro* ou *ex vivo* d'évaluation d'un état d'un épiderme d'une peau sèche comprenant au moins les étapes consistant à :
a) déterminer une teneur d'un polypeptide de séquence d'acides aminés codée par une séquence d'acides nucléiques représentée par une séquence choisie parmi SEQ ID NO 1, SEQ ID NO 2, ou SEQ ID NO 3, un analogue de celles-ci présentant une homologie de séquence d'au moins 85% et une activité biologique de même nature, ou d'acides nucléiques codant pour ledit polypeptide, dans un échantillon d'épiderme, et
b) comparer ladite teneur déterminée à l'étape a) à une valeur de référence, l'état de l'épiderme étant **caractérisé par** une détermination de la teneur déterminée à l'étape a) comme étant de 5 à 10 fois inférieure à la valeur de référence.

2. Procédé selon la revendication précédente, dans laquelle la teneur en polypeptide est déterminée au moyen d'un anticorps se liant spécifiquement à un polypeptide de séquence d'acides aminés représentée par une séquence choisie parmi SEQ ID NO 4, SEQ ID NO 5, SEQ ID NO 6, ou SEQ ID NO 7, ou un analogue de celles-ci présentant une homologie de séquence d'au moins 85% et une activité biologique de même nature.

3. Procédé selon la revendication précédente, dans laquelle l'anticorps se lie à un épitope de séquence d'acides aminés représentée par la séquence SEQ ID NO 7, ou un analogue de celle-ci présentant une homologie de séquence d'au moins 85% et une activité biologique de même nature.

4. Procédé selon l'une quelconque des revendications précédentes, dans laquelle l'état d'un épiderme d'une peau sèche est indicatif d'un éventuel trouble cutané choisi parmi une sécheresse excessive, une xérose, une ichtyose, une atopie, une dermatite atopique, un psoriasis, une hyperkératose, un eczéma, une rosacée, un lichen, un prurit, une couperose, une dermite séborrhéique, une kératodermie palmo-plantaire et un prurigos.

## Patentansprüche

1. In-vitro- oder Ex-vivo-Verfahren zur Bewertung eines Zustands einer Epidermis von trockener Haut umfassend mindestens die folgenden Schritte:
a) Bestimmen eines Gehalts eines Polypeptids einer Aminosäuresequenz, die kodiert wird von einer Sequenz von Nukleinsäuren, die von einer Sequenz dargestellt wird, die ausgewählt wird aus SEQ ID NO 1, SEQ ID NO 2, oder SEQ ID NO 3, einem Analogen davon, das eine Sequenzhomologie von mindestens 85 % und eine biologische Aktivität der gleichen Art aufweist, oder von Nukleinsäuren, die das Polypeptid kodieren, in einer Epidermisprobe, und
b) Vergleichen des in Schritt a) bestimmten Gehalts mit einem Referenzwert, wobei der Zustand der Epidermis **gekennzeichnet ist durch** eine Bestimmung des in Schritt a) bestimmten Gehalts als 5- bis 10-fach kleiner als der Referenzwert.

2. Verfahren nach dem vorangehenden Anspruch, wobei der Gehalt an Polypeptid mittels eines Antikörpers bestimmt wird, der spezifisch an ein Polypeptid der Aminosäuresequenz, die durch eine Sequenz dargestellt ist, die ausgewählt wird aus SEQ ID NO 4, SEQ ID NO 5, SEQ ID NO 6, oder SEQ ID NO 7, oder ein Analog davon bindet, das eine Sequenzhomologie von mindestens 85 % und eine biologische Aktivität der gleichen Art aufweist.

3. Verfahren nach dem vorangehenden Anspruch, wobei der Antikörper an ein Epitop der Aminosäuresequenz, die durch die Sequenz SEQ ID NO 7 dargestellt ist, oder ein Analog davon bindet, das eine Sequenzhomologie von mindestens 85 % und eine biologische Aktivität der gleichen Art aufweist.

4. Verfahren nach einem der vorangehenden Ansprüche, wobei der Zustand einer Epidermis einer trockenen Haut ein Hinweis ist auf eine mögliche Hautirritation, die ausgewählt ist aus übermäßiger Trockenheit, Xerose, Ichtyose, Atopie, atopischer Dermatitis, Psoriasis, Hyperkeratose, Ekzem, Rosacea, Flechte, Puritits, Cuperose, seborrhoischer Dermatitis, palmoplantarer Keratodermie und Prurigo.

## Claims

1. *In vitro* or *ex vivo* method to evaluate the condition of the epidermis of dry skin, comprising at least the steps of:
a) determining the content of a polypeptide having an amino acid sequence encoded by a nucleic acid sequence represented by a sequence selected from among SEQ ID NO 1, SEQ ID NO 2 or SEQ ID NO 3, an analogue thereof having at least 85 % sequence homology and biological activity of same type, or of nucleic acids encoding said polypeptide, in an epidermis sample; and
b) comparing said content determined at step a) with a reference value, the condition of the epidermis being **characterized by** determining the content determined at step a) as being 5 to 10 times lower than the reference value.

2. The method according to the preceding claim, wherein the polypeptide content is determined by means of an antibody specifically binding to a polypeptide having an amino acid sequence represented by a sequence selected from among SEQ ID NO 4, SEQ ID NO 5, SEQ ID NO 6 or SEQ ID NO 7, or an analogue thereof having at least 85 % sequence homology and biological activity of same type.

3. The method according to the preceding claim, wherein the antibody binds to an epitope having an amino acid sequence represented by sequence SEQ ID NO 7, or an analogue thereof having at least 85 % sequence homology and biological activity of same type.

4. The method according to any of the preceding claims, wherein the condition of the epidermis of dry skin is indicative of a possible cutaneous disorder selected from among excessive dryness, xerosis, ichthyosis, atopy, atopic dermatitis, psoriasis, hyperkeratosis, eczema, rosacea, lichen, pruritus, couperose, seborrheic dermatitis, palmoplantar keratoderma and prurigo.
